Europäisches Patentamt

European Patent Office

Office européen des brevets

(19)

(11) Publication number: **0 207 170**

**A1**

(12)

# EUROPEAN PATENT APPLICATION
published in accordance with Art. 158(3) EPC

(21) Application number: **86900275.8**

(22) Date of filing: **26.12.85**

Data of the international application taken as a basis:

(86) International application number:
**PCT/JP85/00718**

(87) International publication number:
**WO86/04092 (17.07.86 86/17)**

(51) Int. Cl.⁴: **C 12 P 21/00**
**C 12 N 15/00, G 01 N 33/574**
**A 61 K 39/395**
**//(C12P21/00, C12R1:91),**
**(C12N15/00, C12R1:91)**

(30) Priority: **28.12.84 JP 281825/84**

(43) Date of publication of application:
**07.01.87 Bulletin 87/2**

(84) Designated Contracting States:
**AT BE CH DE FR GB IT LI LU NL SE**

(71) Applicant: **TAISHO PHARMACEUTICAL CO. LTD**
**24-1 Takata 3-chome Toshima-ku**
**Tokyo 171(JP)**

(72) Inventor: **OHNO, Tsuneya**
**5-15, Fukazawa 2-chome**
**Setagaya-ku Tokyo 158(JP)**

(74) Representative: **Kraus, Walter, Dr. et al,**
**Patentanwälte Kraus, Weisert & Partner**
**Thomas-Wimmer-Ring 15**
**D-8000 München 22(DE)**

(54) **PROCESS FOR PREPARING HUMAN CANCER-SPECIFIC MONOCLONAL ANTIBODY.**

(57) A process for preparing a human cancer-specific monoclonal antibody, which comprises centrifuging a homogenized human cancer cell mixture, subjecting the supernatant to centrifugation in a discontinuous sucrose density gradient solution to collect a fraction gathering on the discontinuous plane, superposing the fraction on a continuous sugar density gradient solution, conducting ultracentrifugation to separate a fraction of 1.191 ρ to 1.210 ρ in density rich in a cancer-specific antigen, immunizing animals with this antigen, preparing a fused cell of the immunized spleen cell and myeloma cell, screening hybridoma capable of yielding the intended antibody, conducting a cloning procedure, and culturing the hybridoma. The resulting monoclonal antibody can provide the doctor with means for discovering cancer at its early stage, instructions on diagnosis and treatment of cancer, and means for monitoring recrudescence of cancer.

## SPECIFICATION

## PROCESS FOR PRODUCING MONOCLONAL ANTIBODIES
## SPECIFIC TO HUMAN CANCER

### FIELD OF TECHNOLOGY

This invention relates to a process for producing monoclonal antibodies specific to human cancer, and more specifically, to a process for producing monoclonal antibodies which react with human cancer tissues and the blood and body fluid components of cancer patients.

### BACKGROUND TECHNOLOGY

CEA (carcinoembryonic antigen) has previously been used for diagnosis of cancer [Gold et al., J. Exp. Med., 121, 439 (1965), J. Exp. Med., 122, 467 (1967)]. CEA is a cancer-related antigen whose production increases markedly in various tumor tissues. Changes in the concentration of CEA in the blood have been used as a tumor marker which well reflects the degree of growth of a tumor tissue.

The following references may be cited with regard to the production of monoclonal antibodies to CEA by a cell fusion method.

R. S. Accolla et al., Proc. Natl. Acad. Sci., U.S.A., 77, 563 (1980),

K. F. Mitchell, Cancer Immunol. Immunother., 10, 1 (1980),

G. T., Rogers, et al., Br. J. Cancer, 43, 1 (1981), and H. Z. Kupchik et al., Cancer Res., 41, 3306 (1981).

However, CEA has a low ratio of capturing cancer. It has little specificity for a particular type of cancer, and is detected also in heavy smokers and fetuses. Hence, although CEA may become a measure of therapy, it raises a problem in utilization for cancer diagnosis.

The present invention relates to a process for producing cancer-specific monoclonal antibodies free from such a problem. The use of the monoclonal antibodies obtained by this invention can provide physicians with guidelines for the early discovery, diagnosis, treatment, etc. of cancer.

With regard to the method for producing monoclonal antibodies, Köhler et al. showed that by fusing spleen cells prepared from immunized mice with mouse myeloma cells, a cell line capable of continuously producing a homogeneous monoclonal antibody can be obtained [Köhler et al., Nature, 256, 495 - 497 (1975), Eur. J. Immunol., 6, 511 - 519 (1976)]. This technique comprises fusing a B-lymphoid cell secreting an antibody with a tumor cell, and the resulting hybridoma cell has the natures of the two cells, namely the ability of the tumor cell to proliferate unlimitedly and the ability of the B-lymphoid cell to produce the particular antibody.

Culturing of this hybridoma made it possible to obtain the particular homogeneous antibody permanently. Since then, various hybridomas were prepared, and much research work has been done on the utilization of monoclonal antibodies produced from these hybridomas. In particular, much interest has been aroused in the utilization of these monoclonal antibodies in the field of the diagnosis and treatment of human cancer.

Monoclonal antibodies specific to human cancer which were proposed in an attempt to solve the problem of CEA mentioned above also have a problem. That problem is human cancer specific antigens, the essential material, cannot be obtained in good purity.

The prior art has no method of preparing human cancer specific antigens which are suitable for the production of monoclonal antibodies and can be applied generally to human cancer. Heretofore, cancer cells have directly been used for the immunization of animals in order to produce monoclonal antibodies to be used for the diagnosis and treatment of human cancer. However, since innumerable antigen determinants exist on the surfaces of the cancer cells, there are innumerable monoclonal antibodies derived from the individual antigen determinants. Because most of these monoclonal antibodies are common in the antigen determinants of normal cells, the production of monoclonal antibodies which specifically react with cancer cells requires the steps of obtaining very many hybridomas and screening them for those hybridomas which produce the desired antibodies.

## DISCLOSURE OF THE INVENTION

The present inventors have undertaken research in order to solve this problem, and have discovered cancer specific antigens useful for obtaining hybridomas which produce the desired monoclonal antibodies very efficiently with little effort, and a process for producing the monoclonal antibodies. This discovery has let to the present invention. The present invention has now offered a complete solution to the prior problem the solution of which has required a great deal of haphazard efforts with anxiety as to whether such efforts will result in obtaining the desired hybridoma.

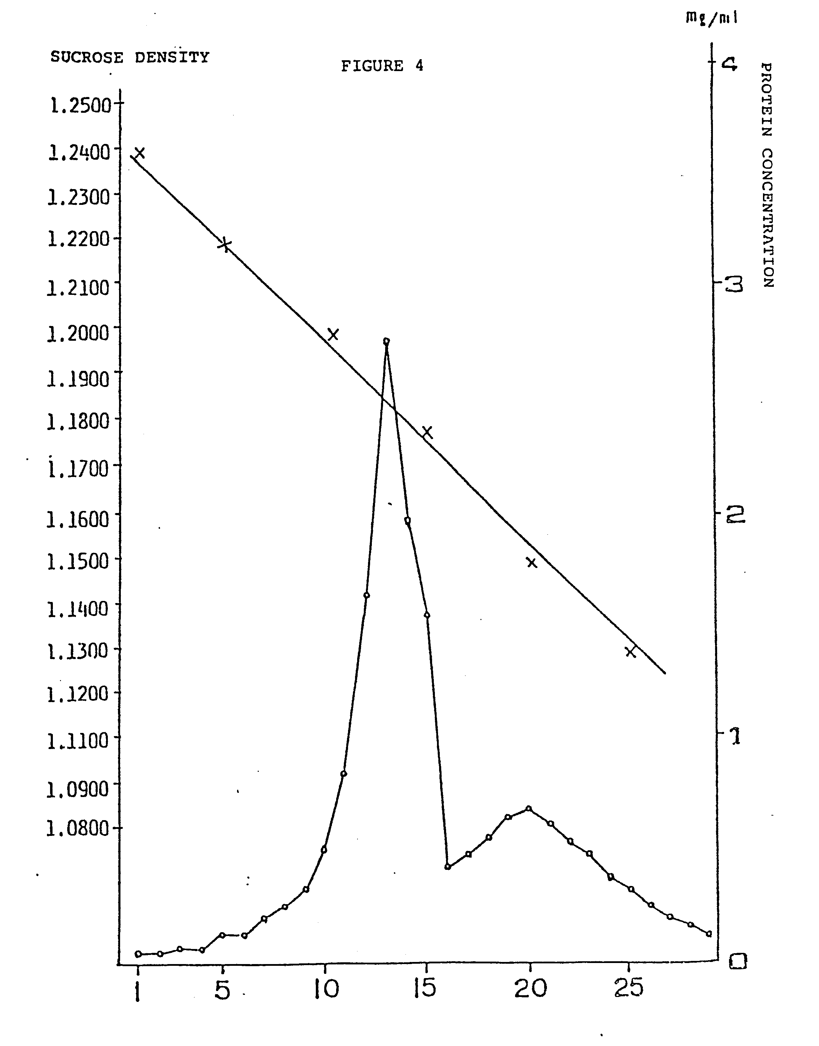

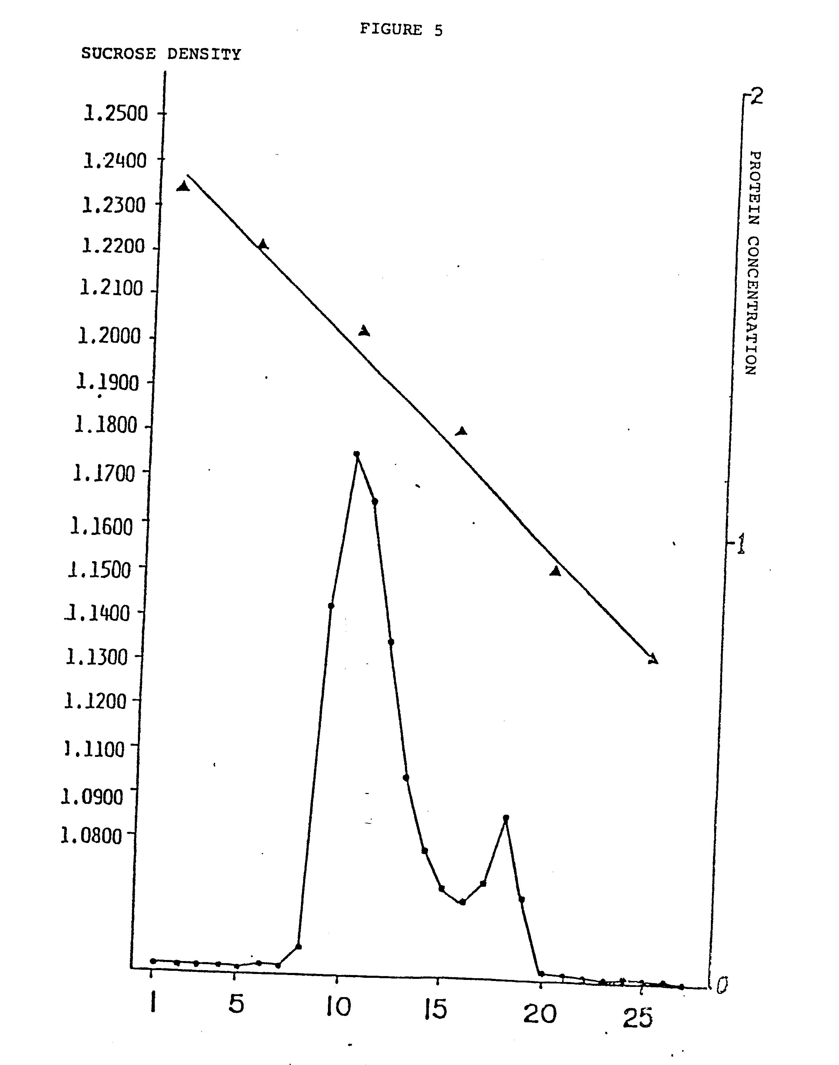

The present inventors worked on the basis of the thought that to produce the desired antibody, the types of antigen substances administered to animals to be immunized should be better as small as possible in number. As a result, it has been found that the membrane components of a normal cell and the membrane components of a cancer cell differ from each other in density gradient centrifugal fractionation pattern. When the membrane components of the normal or cancer cells are centrifuged in a sucrose continuous density gradient buffer, fractions having three peaks based on the densities of the membrane components are observed (designated HR, MR and LR in order of decreasing density), and among them, HR, i.e. a fraction having a heavy membrane component is seen to exist in a larger amount in the membrane components of the cancer cell than in the membrane components of the normal cell (see Figures 4 and 5). Since this increased HR is considered to contain a component inherent to cancer which has formed as a result of malignant alteration of the normal cell, HR is separated and used as an antigen in producing a hybridoma cell between a mouse spleen cell and a mouse tumor cell. By using the resulting hybridoma, the desired monoclonal antibody is produced easily with good efficiency. Table 1 below gives data which clarify this.

Specifically, the present invention is a process for producing a human cancer specific monoclonal antibody, which comprises centrifuging a finely crushed homogenized mixture of human cancer cells, centrifuging the supernatant in a discontinuous sucrose density gradient solution, collecting fractions which gather on the discontinuous phase of the density gradient, superimposing the fractions on a continuous sucrose density gradient solution, ultracentrifuging them to separate fractions rich in a cancer specific antigen having a density of 1.191 $\rho$ to 1.210 $\rho$, immunizing an animal with the fractions as an antigen, preparing fused cells between spleen cells of the immunized animal and myeloma cells, screening the resulting hybridoma cells to select those hybridoma cells which produce the desired antibody, cloning the selected hybridoma cells, and then culturing the clones.

I has been made clear that when the carbohydrate portion of a glycoprotein in a mebmrance component is removed by decomposing it with a mixture of various carbohydrases (such as mannosidase, glucosidase, galactosidase, fucosidase, xylosidase, N-acetylglucosidase and N-acetylgalactosidase), the specificity of the membrane component as a cancer specific antigen increases. It is very interesting that the above two processes for producing antigens can be used to produce antigens specific to various types of human caner in common, and the antigens becomes specific to a cancer type according to the type of cancer used for the isolation of the antigens.

Table 1

| Hybridoma / Immunizing antigen | Breast cancer | | Gastric cancer | | | | Human gastric cancer cell line intact cell | | |
|---|---|---|---|---|---|---|---|---|---|
| | HR | MR | HR | | MR | | KATOIII *5 | MKN28 *6 | MKN45 *7 |
| *1 Total number | 2680 | 3210 | 7680 | 2069 | 5680 | 897 | 1700 | 2800 | 1250 |
| *2 ELISA (+) | 182 | 102 | 268 | 128 | 122 | 98 | 193 | 138 | 43 |
| *3 Cell staining | 32 | 11 | 68 | 28 | 21 | 11 | 21 | 18 | 7 |
| *4 Cancer specific  Cancer (+) / Normal (−) | 2 | 0 | 2 | 5 | 0 | 0 | 0 | 0 | 0 |

*1: Hybridomas obtained by using HR or MR of breast cancer or gastric cancer as immunizing antigens. The cell lines were obtained by using the cells directly as immunizing antigens.

*2: The number of hybridomas which showed a positive result in ELISA conducted by using the sonicate of cancer cells as an antigen.

*3: The number of hybridomas which showed a positive result in staining of cancer tissue cells.

*4: The number of hybridomas which did not react with normal cells but reacted only with the cancer cells.

*5: Cell line isolated and established from human gastric cancer (obtained from Prof. Watanabe, Niigata University, First Pathology Department)

*6: Cell line isolated and established from human gastric cancer (obtained from Prof. Watanabe, Niigata University, First Pathology Department)

*7: Cell line isolated and established from human gastric cancer (obtained from Prof. Watanabe, Niigata University, First Pathology Department)

The process of this invention will be described below in detail.

Human cancer tissues are cut into pieces under ice cooling, and homogenized while slowly adding suitable buffer such as a 5% sucrose-containing Tris-HCl buffer.

The homogenate is then centrifuged for about 10 minutes at about 650 x g to about 1,500 x g. The pellets are removed and the supernatant is centrifuged for about 10 minutes at 100,000 g to precipitate the nucleoli, mitochondria, etc. The supernatant is then superimposed on a discontinuous sucrose density gradient prepared from a 20% - 28%, usually about 25%, sucrose-containing buffer and a 45% - 60%, usually about 50%, sucrose-containing buffer, and centrifuged at about 60,000 x g to about 200,000 x g, for at least about 60 minutes, usualy for about 90 minutes. Fractions containing the desired antigen gather on the discontinuous phase of the density gradient. The fractions are collected and adjusted to an about 20% sucrose concentration with a buffer, and then superimposed on an about 20% - about 50% continuous sucrose density gradient using a buffer such as Tris-HCl buffer, and centrifuged at about 60,000 x g to about 200,000 x g, for at least 4 hours. The desired antigen exists in fractions having an aqueous solution density of 1.191 ρ to 1.210 ρ (calculated as 41.6% -45.2% as the carbohydrate concentration).

The fractions are collected, diluted with an HCl buffer to a carbohydrate concentration of not more than about 20%, and centrifuged at 60,000 x g to 200,000 x g, for at least about 60 minutes, usually for about 90 minutes to give the desired antigen as pellets (this antigen is provisionally named HR-300).

To obtain an antigen having higher cancer specificity, the following procedure is taken. HR-300 is subjected to an operation of removing fats by using a cooled organic solvent such as acetone, ether, chloroform or methanol, and then subjected to glycolysis using a suitable carbohydrase. Glycolysis is carried out in a suitable buffer such as 0.05M sodium citrate buffer. As the carbohydrase, a mixture of alpha-glucosidase, beta-glucosidase, alpha-mannosidase, beta-mannosidase, alpha-galactosidase, beta-galactosidase, alpha-L-fucosidase, beta-xylosidase, alpha-N-acetylglucosaminidase and beta-N-acetylgalactosaminidase is used. HR-300 from which lipids and the carbohydrate portion have been removed is centrifuged at about 60,000 x g to 200,000 x g, for at least 60 minutes, usually for 90 minutes, and collected as pellets which are named HR-100.

The process for producing a monoclonal antibody will be described.

The antigen in accordance with this invention is administered to an animal several times together with a suitable adjuvant such as Freund's complete adjuvant to produce an antibody. For example, when the antigen in accordance with this invention is intraperitoneally or subcutaneously administered, for example, to BALB/C mice in an amount of 1 to 60 micrograms per mouse and the antigen without an adjuvant is intravenously administered to the animals in a dose of 1 to 20 micrograms one to two weeks later, the animals are immunized.

To obtain antibody-producing cells from the immunized animals, it is usually practical to utilize the spleen cells. For example, the spleen of the BALB/C mouse is extracted three days after the administration of the antigen without an adjuvant and with cooling, cut into small pieces in RPMI-1640 or PBS buffer, filtered through a stainless steel mesh, and adjusted to a suitable cell number with the above buffer.

The myeloma cell lines used for cell fusion are, for example, those derived from BALB/C mouse MOPC 21, such as P3-NSI/1-Ag4-1 (generally called NS-1) described, for example, by Kohler et al. in Eur. J. Immunòl., Vol. 6, 292 - 295 (1976). Cell lines of this type are 8-azaguanine-resistant and lack hypoxanthine-guanine phosphoribosyl transferase, and therefor cannot survive in HAT medium (medium containing hypoxanthine-aminopterin-thymidine).

Polyethylene glycol having an average molecular weight of 1000 to 6000 is suitable as a cell fusion agent, and polyethylene glycol 1500 is especially desirable. The concentration of the cell fusion agent used is 40% to 50% depending upon its molecular weight. When polyethylene glycol 1500 is used, it is adjusted to a concentration of 50 % by RPMI-1640. In performing cell fusion, first the antibody-producing cells and myeloma cells are washed with RPMI-1640 and mixed in a ratio of from 4:1 to 10:1, and centrifuged for about 5 minutes at 800g. The medium is decanted, and the cell
fusion agent is slowly added to the resulting pellets with
stirring over 1 minute in an amount of about 1 ml (37 °C) per
1 to 2 x $10^6$ cells. Further with continued stirring, the
mixture is suspended gradually in RPMI-1640 (37 °C; 5 to 10
ml) over 5 to 10 minutes. After centrifugation at 400 to 600
g for 5 minutes, the supernatant is decanted, and adjusted to
a cell number of 1 to 2 x $10^6$/ml with RPMI-1640 containing 15%
horse serum. Hybridomas are suitably isolated by culturing in
HAT medium (HAT selection). The cell suspension after the
fusion was placed in a 96-well plate for tissue culture in an
amount of 0.1 ml per well, and cultivated at 37 °C for 24
hours. Half of the culture broth is replaced by HAT medium.
Thereafter, every 1 to 4 days, half of the culture fluid is
replaced by HAT medium, and the culturing is continued for
about 3 weeks. All the parental cells die, and only the fused
cells grow. Thereafter, the fused cells are cultured in RPMI-
1640 containing 15% horse serum to produce antibodies. By a
suitable method such as enzyme linked immunosorbent assay
(ELISA), it is determined whether the desired antibody is
produced by these hybridomas.

The antigen is selected according to the desired antibody.
For selection of antibodies specific to breast cancer,
screening is carried out by using cell lines MCF7 and T-47D
isolated from human breast cancer tissues, for example. For
selection of antibodies specific to gastric cancer, screening
is carried out by using cell line KATO III isolated from human gastric cancer tissues. As a control, the cell membrane components of cell line 3T3 derived from normal mouse cell are used. The cell components are prepared by the method of preparing the antigen HR-300 in accordance with this invention.

First, the above antigen is prepared, diluted to 3 to 5 micrograms/ml with a suitable buffer such as sodium carbonate buffer (pH 9.5), and coated on the individual wells of a 96-well microtiter plate. After standing overnight at 4 °C, the plate is well washed with phosphate buffer (PBS) containing polyoxyethylene sorbitan monolaurate (Tween 20), and subjected to a blocking operation using, for example, horse serum. Then, the above hybridoma culture fluid is added in an amount of 0.1 ml for each well, and the plate is left to stand at 37 °C for 5 to 6 hours.

The culture fluid is then decanted, and well washed with the PBS buffer. Then, 1% normal goat serum is added. The mixture is left to stand at 37 °C for about 1 hour to block non-specific adsorption. It is then washed with the PBS buffer, and peroxidase-conjugated IgG of goat anti-mouse globuline is added. The mixture is incubated at 37 °C for about 4 hours, and then washed with the PBS buffer.

A sodium citrate buffer containing hydrogen peroxide and o-phenylenediamine dihydrochloride as a substrate are added, and the absorbance is measured 30 minutes later. A culture of cells which do not produce antibodies is used as a control, and by a difference of the absorbance from that in each well, the well which showed a significant color reaction is rated as being positive.

The desired antibody is positive against MCF7 and T-47D in the case of breast cancer; and is positive against KATO III in the case of gastric cancer. In both cases, the antibody is negative against mouse 3T3. Furthermore, it is determined that the antibody has specificity for a pathological slice, and hybridomas which produce it are obtained.

The physiological slice is prepared from a formalin-fixed paraffin slice of a human cancer tissue.

The paraffin is removed by using xylene, and the residue is treated stepwise with 100% ethanol to 70% ethanol and finally well washed with PBS buffer. Hyaluronidase is caused to act on the slice at 37 °C for 1 hour, washed with PBS buffer, and then incubated with normal goat serum containing trasylol at 37 °C for about 10 minutes. The slice is washed with PBS buffer and reacted at 37 °C for 20 minutes with the monoclonal antibody obtained after the aforesaid screening. After washing with PBS buffer, the peroxidase-conjugated IgG fraction of goat anti-mouse globulins is added, and the mixture is incubated at room temperature for 30 minutes. The slice is further washed with PBS buffer and immersed for 10 minutes in PBS buffer saturated with DAD (3,3'-diaminobenzidine) containing hydrogen peroxide. The slice washed with PBS is stained with methylene blue in a customary manner, dehydrated with ethanol, and washed with xylene to form a pathological slice. The slice is microscopically observed.

The desired hybridoma capable of producing a cancer specific antibody is fractionated and cultured by suitable methods to form a monoclonal cell strain derived from a single cell (cloning). The limiting dilution method is general.

For example, thymus cells or spleen cells of BALB/C mice are prepared as a feeder so that they amount to 1 to 5 x $10^7$/ml, and poured dividedly into the wells of a 96-well tissue culture plate in an amount of 0.1 ml per well. A dilute suspension of the screened antibody-producing hybridoma is prepared, and poured into the individual wells of the plate.

Suspending of the feeder and the hybridoma is carried out by using HAT medium or RPMI-1640 medium containing horse or fetal calf serum, and one hybridoma cell is placed in each well. The addition of the feeder is not essential. Then, the plate is incubated at 37 °C in a 5% $CO_2$ culture device, and 1 to 2 weeks later, clones grow. By a microscope, those wells in which only one clone grows per well are selected and analyzed by the aforesaid screening method. Thus, clone which produce the desired antibody are selected.

The screened hybridomas can be caused to produce the antibody both _in vitro_ and _in vivo_ cultivations.

The _in vitro_ cultivation may be carried out by cultivating the hydbridomas in a suitable nutrient medium, for example RPMI-1640 medium supplemented with fetal calf serum or horse serum, and this is suitable for obtaining pure monoclonal antibodies not containing other immunoglobulins.  For the production of large amounts of monoclonal antibodies, in vivo cultivation is convenient.

For example, it is carried out by the following method. 2,6,10,14-tetramethylpentadecane (pristan) is intraperitoneally administered to BALB/C mice in an amount of 0.5 ml.  After the lapse of 2 to 20 days, the screened hybridomas (1 to 5 x $10^6$ cells) are administered intraperitoneally.  Hybridomas which proliferate and settled 2 to 3 weeks later are ascites tumorigenic cells.  By intraperitoneally administering the cells to the required number of mice, ascites containing the desired monoclonal antibody can be continuously obtained 2 to 3 weeks later and thereafter.  The specific antibody activity is confirmed by a suitable method, and an authentic sample of high purity can be obtained by suitable means such as salting-out with ammonium sulfate, ion exchange on DEAE cellulose, gel filtration and affinity chromatography.

As shown in Examples given hereinafter, the monoclonal antibodies in accordance with this invention show an antigen-antibody reaction specific to the cancers and by utilizing this character, a diagnostic agent capable of detecting cancer with very high sensitivity is provided.

Now, a diagnostic agent containing monolonal antibodies will be described taking a human breast cancer-specific monoclonal antibodies as an example.

A substance which reacts with the monoclonal antibodies of this invention (the monoclonal antibodies derived from cancer-specific antigens HRBC-300 and HRBC-100 of this invention prepared from human breast cancer tissues) is observed in culture supernatants of cell lines MCF7 (ATCC deposit number: HTB-22) and T-47D (ATCC deposit number: HTB-133) isolated from human breast cancer. This suggests the possibility that the above substance which reacts with the monoclonal antibodies also exists in the serum of a cancer patient. Extensive investigations have led to the accomplishment of the present invention.

The diagnostic agent may be any which utilizes an immunological testing method if it contains the monoclonal antibody of this invention.

Testing methods, that can be used include, for example, complement fixation reaction (CF), immune adherence hemagglutination (IAHA), reverse passive hemagglutination (R-PHA), radioimmunoassay (RIA), enzyme immunoassay (EIA), and enzyme linked immunosorbent assay (ELISA).

The antibody of the invention may be prepared in a suitable form according to the testing methods.

When the reverse passive hemagglutination method is utilized, the antibody of this invention is bounded to fine particles by using glutaraldehyde, tannic acid, chromium chloride, etc. Suitable fine particles include, for example, red blood cells of mammals and birds, a polystyrene latex, and vinyl chloride resin and glass beads.

When radioimmunoassay is utilized, the antibody of this invention is labelled with radioisotopes such as $^{131}I$, $^{125}I$, $^{14}C$ and $^3H$.

When the enzyme linked immunosorbent assay is used, the enzyme is bounded to the antibody of this invention by using glutaraldehyde. Usable enzymes include, for example, peroxidase, glucoseoxidase, alkaline phosphatase and beta-galactosidase.

The monoclonal antibody obtained by using the cancer-specific antigen fraction of this invention and its glycosidase-treated product have cancer specificity for the type of the cancer used as a raw material and permits the preparation of a diagnostic agent specific to the cancer used. In Examples given below, antigens were prepared by using breast cancer tissue, gastric cancer tissue, or their cultivated cells, and monoclonal antibodies were prepared. From them, diagnostic agents for gastric and breast cancers were prepared.

For example, the monoclonal antibodies prepared in Examples 3 and 4 (an antibody obtained by using the HR fraction and an antibody obtained by glycosidase treatment of the HR fraction) each recognized antigen determinants on the same molecule of the constituent substances of the breast cancer and gastric cancer specific antigens. Accordingly, by utilizing the two monoclonal antibodies in the so-called "sandwich method", serological diagnostic agents of breast and gastric cancers were prepared.

The "sandwich method" is an antigen analysis method using an antigen having two antigen determinants at spatially different positions on the same molecule and two antibodies corresponding to the two determinants. Specifically, an antibody (a) having low antigen specificity and (b) an antibody having higher specificity and labelled with a substance which can be an index for analysis are used.

Specifically, this method permits the detection of the intended antigen by coating and adsorbing the antibody (a) on glass beads, a plastic plate, etc., contacting it with a liquid containing the intended antigen to collect this antigen and similar antigens, and finally contacting it with the labelled antibody (b) and analyzing the label. The advantage of this method is that a monoclonal antibody having very high antigen specificity but lacking sensitivity can be rationally utilized.

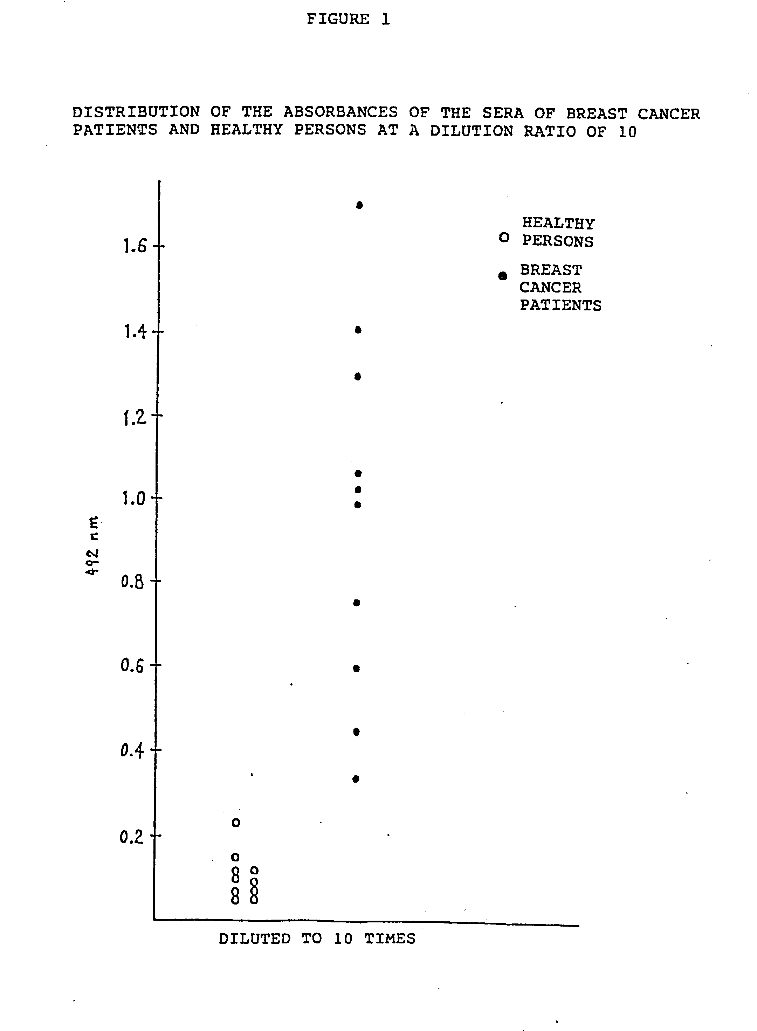

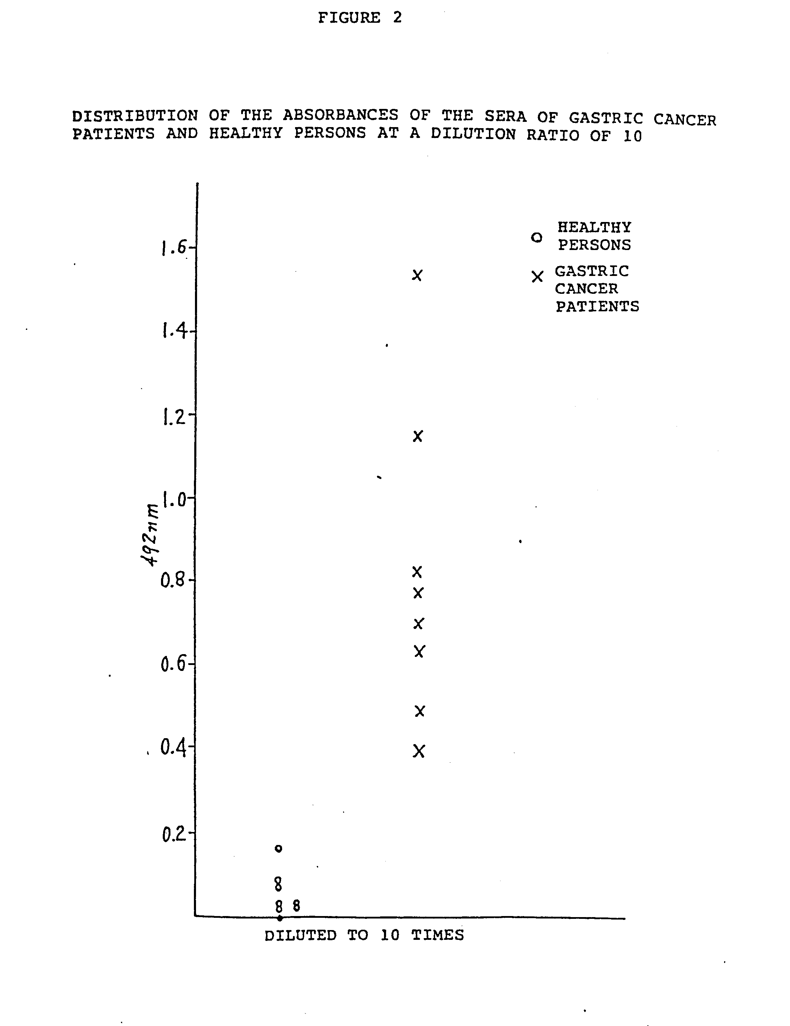

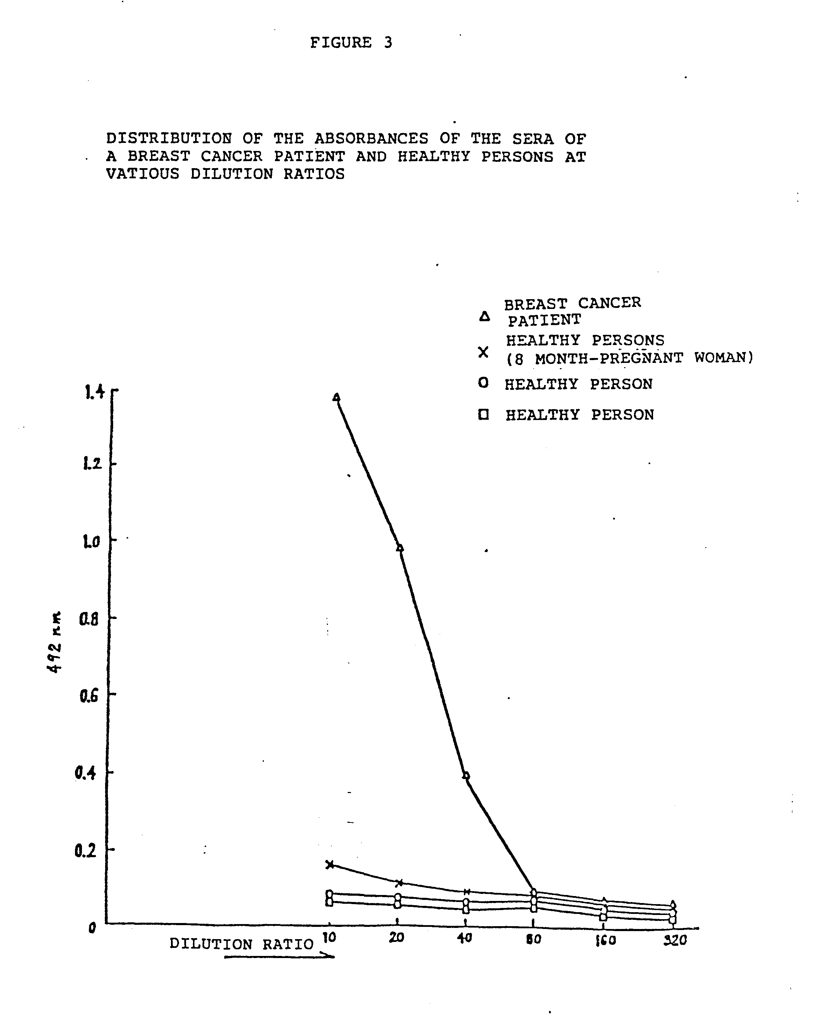

This invention will be specifically described. The monoclonal antibody to the HR fraction of Example 3 is diluted to a concentration of 3 to 20 micrograms/ml with 0.05M sodium carbonate buffer (pH 9.5) or a suitable buffer such as PBS buffer [140mM NaCl, 13mM $Na_2HPO_4$, 2mM $NaH_2PO_4$ (pH 7.6)]. The dilution is poured individually into the wells of an untreated microtiter plate in an amount of 0.1 to 0.2 ml per well, and left to stand overnight at a low temperature. The plate is then well washed with a suitable buffer such as PBS buffer containing 0.05% of polyoxyethylene sorbitan monolaurate (Tween 20). An assay sample to be analyzed (such as human serum or body fluids) is diluted stepwise, added in a fixed amount of 0.1 to 0.2 ml and incubated at 37 °C for 3 to 6 hours.  Thereafter, the individual wells of the plate are well washed with the PBS buffer containing Tween 20.  PBS buffer containing 3 to 5 micrograms/ml of each of monoclonal antibodies to HRBC-100 or HRGC-100 to be described, which has previously been labelled with horseradish peroxidase by the method of Nakane et al. (P. K. Nakane and A. Kawaoi, J. Histochem. Cytochem., 22, 1084, 1974), is added in a fixed amount of 0.1 to 0.2 ml and incubated at 37 °C for 3 to 6 hours.  Then, the plate is well washed with PBS buffer containing Tween.  Sodium citrate buffer (pH 5.6) containing hydrogen peroxide and OPD (ortho-phenylenediamine dihydrochloride) as a substrate are added, and after 30 minutes at room temperature, 50 microliters of 0.4M sulfuric acid is added and mixed.  The absorbance of each well is then measured, and the concentration of the antigen is calculated from the difference in absorbance between 492 nm and 610 nm. To demonstrate the utility of this diagnostic agent, cancer is diagnosed by using the serum of a cancer patient.  For example, by using serum samples of 10 breast cancer patients, 8 gastric cancer patients and 10 healthy persons, the absorbances are measured by the method described in Example 5 given hereinafter.  The results are shown in Figures 1 and 2 prepared by plotting the absorbances at a wavelength of 492 nm using the serum samples diluted to 10 times.  The serum samples from the breast cancer patients and the gastric cancer patients both show an absorbance of at least 0.3, whereas the average absorbance of the serum samples taken from the healthy persons is not more than 0.1.  This shows that large amounts of the breast cancer specific antigen and the gastric cancer-specific antigen exist in the blood of the breast cancer

patients and the gastric cancer patients respectively, but are hardly present in the blood of the healthy persons.  Only one (a pregnant woman in the 8th months of pregnancy) out of the healthy persons showed a slightly high absorbance value of 0.23.  Figure 3 is a graphic representation of the absorbances of serum samples taken from a breast cancer patient, the above pregnant woman and other two healthy persons at various dilution ratios.  As can be seen from the foregoing, breast cancer and gastric cancer can be clearly and accurately diagnosed by examining the absorbances of sera at a fixed dilution ratio. Thus, the utility of the diagnostic agent is demonstrated.


                    Brief Description of the Drawings

        Figure 1 is a diagram showing the distribution of the absorbances of serum samples taken from breast cancer patients and healthy persons and diluted to 10 times; Figure 2 is a diagram showing the distribution of the absorbances of serum samples taken from gastric cancer patients and healthy persons and diluted to 10 times; Figure 3 is a diagram showning the absorbances of serum samples taken from a breast cancer patient and healthy persons and diluted at various ratios;

Figure 4 is a graph showing the relation among fractions separated for normal cell membrane components by sucrose density gradient centrifugation, sucrose densities and protein concentrations (in the figure, O-O represents protein

concentration, and X-X, sucrose concentration); and Figure 5 is a graph showing the relation among fractions separated from gastric cancer cell membrane components by sucrose density gradient centrifugation, sucrose densities and protein concentrations (in the figure, ●-● represents protein concentration, and ▲-▲ sucrose concentration).


Best Mode of Practicing the Invention The present invention is illustrated below by Examples.

Example 1

Production of antigens HRBC-300 and HRGC-300

Human breast cancer tissues and human gastric cancer tissues were each cut into pieces under ice cooling, and homogenized in TNE buffer [0.01M Tris-HCl (pH 7.5), 0.15M NaCl, 0.003M EDTA (pH 8.0)] containing 5% (W/W) sucrose by using a cell crushing device. At this time, the Tris-HCl

buffer was added at a rate of 4 ml per gram of the cancer tissues, and the cancer tissues were crushed for 15 seconds and then cooled for 30 seconds. This operation was repeated four times. Then, the homogenate was centrifuged at 650g for 10 minutes. The supernatant (sup-1) was further centrifuged at 10,000g for 10 minutes. As a result, the nucleoli, mitochondria, etc. precipitated, and a supernatant (sup-2) containing the desired antigen component was obtained.

Then, a discontinuous density gradient was prepared from 8 ml of TNE buffer containing 20% (W/W) sucrose and 6 ml of TNE buffer containing 50% (W/W) sucrose, and 25 ml of the above supernatant (sup-2) was superimposed and centrifuged for 90 minutes at 100,000g by a swinging bucket (SW 27). Fractions containing the desired antigen were obtained in the interface between the two buffers having different densities. Thereafter, a continuous density gradient was prepared by using 15 ml of TNE buffer containing 20% (W/W) sucrose and 15 ml of TNE buffer containing 50% (W/W) sucrose. Ten milliliters of the sample obtained in the density interface in the above discontinuous density gradient centrifugation and adjusted the concentration of sucrose to 20 % using TNE buffer was superimposed on the continuous density gradient. The sample was centrifuged at 100,000g for 12 hours using SW27. After centrifugation, 32-33 fractions each in a volume of 1.2 ml were separated from the bottom of the swinging bucket. The densities of sucrose were measured, and those fractions which had a density of 1.191 $\rho$ to 1.210 $\rho$ (sucrose concentration 41.6% to 45.2%) were collected, adjusted to a sucrose concentration of not more than 20% with TNE buffer, and centrifuged at 100,000g for 90 minutes. By collecting the pellets, the desired antigens were obtained (the antigens are named HRBC-300 and HRGC-300).

Example 2

2-1. Production of antigens HRBC-100 and HRGC-100

Each of the antigens HRBC-300 and HRGC-300 obtained in Example 1 was defatted with a suitable amount of cooled acetone, and then reacted with 50 micrograms, per 500 micrograms of HRBC-300 or HRGC-300, of each of various carbohydrases (alpha-mannosidase 166 units/mg, beta- mannosidase 166 units/mg, alpha-glucosidase 3.2 units/mg, beta-glucosidase 20 units/mg, alpha-galactosidase 24 units/mg, beta-galactosidase 125 units/mg, alpha-L-fucosidase 58 units/mg, beta-xylosidase 12 units/mg, alpha-N-acetylglucosaminidase 2.5 units/mg, beta-N-acetylgalactosaminidase 46 units/mg; Seikagaku Kogyo Co. Ltd.)

at 37 °C overnight in 0.05M sodium citrate buffer (pH 4.0).

Then, 1M Tris-HCl buffer (pH 7.5) was added to stop the reaction.  To the reaction mixture was added dropwise an equal amount of saturated ammonium sulfate ( pH 7.0), and they were mixed for 30 minutes.  The mixture was centrifuged at 4 °C and 10,000g for 20 minutes to obtain a precipitate.  The precipitate was dissolved in PBS buffer (pH 7.6), and dialyzed against PBS buffer.  As a result, the desired antigens HRBC-100 and HRGC-100 from which carbohydrate components had been removed were obtained.

2-2.    Analysis of the antigens

By taking up the breast cancer antigens (HRBC-300 and HRBC-100) as examples, proteins were quantitatively determined by the Lowry method [O. H. Lowry, N. J. Rosenbrough, A. L.

Farr & R. J. Randall, J. Biol. Chem., 193, 265 (1951)] as follows:-

Two hundred microliters of a sample solution containing 3 to 20 micrograms of proteins was mixed with 1.0 ml of a copper sulfate-tartaric acid-alkali mixture (1% aqueous copper sulfate pentahydrate solution; 1% aqueous potassium tartrate solution; 0.1N NaOH + 2% aqueous $Na_2CO_3$ solution, 1:1:100). The mixture was left to stand at room temperature for 10 minutes, and 0.1 ml of a phenol reagent (x 1/2) was added and mixed. The mixture was left to stand at room temperature for 30 minutes, and its absorbance at 660 m was measured and proteins were quantitatively determined by using BSA as a standard.

Carbohydrates were quantitatively determined by the anthrone method [Lectures in Biochemical Experiments, 4, Chemistry of Carbohydrates (2nd volume), 370 (Tokyo Kagaku Dojin)].

Three hundred microliters of anthrone-sulfuric acid (prepared by dissolving 10 mg of anthrone in 0.25 ml of distilled water, adding 4.75 ml of concentrated sulfuric acid in cold water, and further adding 1 ml of distilled water) was added to 50 microliters of a sample solution containing 1.3 to 2.2 micrograms of carbohydrates. The mixture was heated for 10 minutes in boiling water, and then treated with flowing water to cool it to room temperature. The absorbance of the solution at 620 m was measured, and carbohydrate were quantitatively determined by using soluble starches as a standard. The results are shown in Table 2.

Table 2

| Sample | Proteins (μg/μℓ) | Carbohydrates (μg/μℓ) | Carbohydrate μg/ proteins μg |
|---|---|---|---|
| HRBC-300 | 0.55 | 0.125 | 0.227 |
| HRBC-100 | 2.58 | 0.197 | 0.147 |
| *MRBC | 1.34 | 0.220 | 0.085 |
| **MRBC-glycosidase | 2.12 | 0.168 | 0.079 |

*Glycoprotein components having a low molecular weight obtained during the preparation of HRBC-300.

**Product obtained by treating MR with various carbohydrase.

Example 3

Production of monoclonal antibodies using HRBC-300 and HRGC-300 as immunization antigens

3-1.    Immunization of mouse spleen cells

Fifty micrograms of HRBC-300 or HRGC-300 dissolved in 0.3 mℓ of PBC buffer containing an equivalent weight of Freund's complete adjuvant was administered intraperitoneally to 6-week old BALB/C or NZB mice. One week later, 25 micrograms of HRBC-300 or HRGC-300 dissolved in 0.3 ml of PBS buffer containing an equivalent weight of Freund's incomplete adjuvant was intraperitoneally administered. Furthermore, one week later, 5 micrograms of HRBC-300 or HRGC-300 dissolved in 0.3 ml of PBS buffer was administered through the tail vein. Three days later, the spleen was extracted, washed with PBS buffer, and filtered through a stainless steel mesh to form a suspension of single cells. Then, the number of cells was adjusted to $2 \times 10^6$/ml using PBS buffer.

3-2. <u>Fusion of immunized spleen cells with mouse myeloma cells</u>

The mouse myeloma cells used were derived from BALB/C mouse MOPC21 and called mouse myeloma NS-1, as described, for example, by Kohler in Eur. J. Immunol., <u>6</u>, 292 - 295 (1976). First, the mouse myeloma cells in the logarithmic growth stage cultivated in RPMI-1640 complete medium (glutamine 2mM, sodium pyruvate 1mM, fungizone 0.25 micrograms/ml, streptomycin 50 micrograms/ml, penicillin 50 units/ml, horse serum 15 %) were washed with RPMI-1640-medium free from horse serum, and the number of the cells was adjusted to $5 \times 10^5$/ml using RPMI-1640 medium free from horse serum. Then, 40 ml of a suspension of the immunized mouse spleen cells prepared in Example 3-1 was mixed with 10 ml of the above suspension of the mouse myeloma cells, and the mixture was centrifuged at 800g for 5 minutes to form pellets. The supernatant was completely removed by decantation. To the pellets were gradually added with stirring 1 ml of a solution of polyethylene glycol (PEG-1500) warmed at 37 °C (PEG-1500 solution adjusted to a concentration of 50% by using horse serum-free RPMI-1640). The mixture was stirred for 1 minute, and then 6 ml of horse serum-free RPMI-1640 was gradually added over 6 to 7 minutes with stirring. The mixture was centrifuged at 600g for 5 minutes. The supernatant was removed, and then RPMI-1640 was added to form a cell suspension containing $2 \times 10^6$ cells/ml.

3-3.    <u>HAT selection</u>

The cell suspension prepared in Example 3-2 was dividedly poured into the wells of a 96-well plate for tissue culture in an amount of 0.1 ml per well, and cultured at 37 °C in a 5% $CO_2$ culture device. Twenty-four hours later, 0.1 ml of HAT medium (RPMI-1640 containing hypoxanthine, aminopterin and thymidine; Littlefield, Science, 145, 709 - 710, 1964) was added to the culture fluid in each well, and then 0.1 ml of the culture fluid was removed from each well, and the culture was continued. At 2, 3, 5, 8, 11, 14, 17 and 21 days after the initiation of culturing, the above operation of replacing half of the culture fluid by the HAT medium was repeated. By this HAT selection, the myeloma cells died, and only the fused cells grew. The grown hybridoma cells were cultured in RPMI-1640 containing horse serum, and the culture fluid was used as an analytical sample for enzyme-linked immunosorbent assay (ELISA).

3-4.    ELISA

By the method of Example 1, an antigen was prepared from the cultured cells of each of cell line 3T3 (cell line from normal mouse cells, deposited as CCL92 in ATCC), cell line MCF7 and cell line T-47D (both isolated and established from human breast cancer, deposited as HTB-22 and HTB-133, respectively, in ATCC) and cell line KATO III (isolated and established from human gastric cancer, obtained from Prof. Watanabe of Niigata University), and diluted to a concentration of 4 micrograms/ml with sodium carbonate buffer (0.05 M $NaHCO_3$, 0.05 M $Na_2CO_3$, pH 9.5). The dilution was then dividedly poured into the wells of a 96-well microtiter plate in an amount of 0.1 ml per well, and after sealing its surface, the plate was left to stand overnight at 4 °C. On the next morning, the plate was well washed with PBS buffer containing 0.05% polyoxyethylene sorbitan monolaurate (Tween 20), and 0.1 ml of the hybridoma culture fluid prepared in Example 3-4 was added to each of the wells, and the plate was left to stand at 37 °C for 6 hours. As a control, a culture fluid of cells not producing an antibody was used.

The culture fluid was decanted, washed five times with PBS buffer containing 0.05% Tween, and 0.1 ml of PBS buffer containing 1% normal goat serum was added to each well. After standing at 37 °C for 1 hour, the plate was well washed with PBS buffer containing Tween.

Then, 0.1 ml of a horseradish peroxidase-conjugated IgG fraction of goat anti-mouse globulins from a refrigerated stock, diluted to 1:30 with PBS buffer, was added in an amount of 0.1 ml per well, and incubated at 37 °C for 4 hours. Each of the cells was washed four times with PBS buffer containing Tween 20, and 1/100 volume of 40mM ABTS [diammonium 2,2'-azino-di(3-ethylbenzothiazoline)sulfonate] and 1/100 volume of 0.5% citrate-phosphate buffer (pH 5.8) containing 30% hydrogen peroxide were added as a substrate. Thirty minutes later, the absorbance of each well was measured. Those which showed a color reaction significant over the control were rated "positive". In the case of breast cancer, hybridomas which reacted with HRBC-300, MCF7 but T-47D and did not react with mouse 3T3 were selected.  In the case of gastric cancer, those which reacted with HRGC-300 and KATO III but did not react with mouse 3T3 were selected.  Furthermore, it was confirmed that the selected hybridomas had reaction specificity for a pathological tissue slice.  The selected hybridomas were cloned by the limiting dilution method.

3-5.     Method of staining a tissue slice

A formalin-fixed paraffin slice of human breast or gastric cancer was immersed in xylene to dissolve paraffin, then immersed stepwise in an ethanol solution having a concentration of 100 % to 70 %, and finally washed well with PBS buffer.

Then, 0.15 ml of hyaluronidase (600 units/ml; 0.01m PBS buffer, pH 6.5) was caused to act on the slice at 37 °C for 1 hour.  The slice was washed with PBS buffer, and incubated at 37 °C for 10 minutes with normal goat serum (adjusted to 50% concentration with PBS buffer) containing 0.5 unit/ml of trasylol as a proteinase inhibitor to block non-specific adsorption on the tissue.  The slice was then gently washed with PBS buffer, and incubated at 37 °C for 20 minutes with each of the monoclonal antibodies screened in Example 3-4. After washing three times with PBS buffer, the horseradish peroxidase-conjugated IgG fraction (adjusted to 100 micrograms IgG/ml based on the entire goat serum with PBS buffer) of goat anti-mouse globulins was caused to act at room temperature for 30 minutes.  The slice was further washed with PBS buffer, and then immersed at room temperature for 10 minutes in PBS buffer containing 30% hydrogen peroxide and saturated with DAD (3,3'-diaminobenzidine). Thereafter, the slice was washed with PBS buffer, stained with methylene blue, stepwise dehydrated with 70%-100% ethanol, and finally washed with xylene to prepare a pathological slice.

3-6. Limiting dilution method

The hybridoma obtained by screening in Example 3-4 was cultured in RPMI-1640 containing horse serum, and then diluted with RPMI-1640 containing horse serum to a cell number of 100/ml, 50/ml, 10/ml and 5/ml, respectively. Each of the suspensions was dividedly poured into the wells of a 96-well plate for tissue culture in an amount of 0.1 ml. The culture fluids in the wells in which cell proliferation was observed were examined for antibody production in accordance with ELISA in Example 3-4. This operation was repeated twice to obtain monoclonal cells.

3-7. Mass production of antibodies

0.5 ml of pristan (2,6,10,14-tetramethylpentadecane) was intraperitoneally administered to BALB/C mice, and 20 days later, the monoclonal hybridoma cells obtained in Example 3-6 $(2 \times 10^6)$ were inoculated in the animals. Ten to 20 days later, ascites tumor developed, and ascites tumor cells were obtained. About $2 \times 10^6$ ascites tumor cells were intraperitoneally administered to other BALB/C mice. Two to three weeks later, ascites containing the desired monoclonal antibody was obtained.

With regard to HRBC-100 and HRGC-100, hybridomas producing cancer specific monoclonal antibodies and the monoclonal antibodies produced thereby were obtained by the steps of Example 3.

The monoclonal antibodies were purified and analyzed, and it was found that HRBC-300 ($IgG_1$), HRBC-100 ($IgG_1$), and HRGC-100 ($IgG_{2a}$) had a molecular weight of 160,000 daltones, and HRGC (IgM) had a molecular weight of 900,000 daltones.

3-8.    Purification of the antibodies

An equivalent weight of PBS buffer was added to 10 ml of the ascites containing antibodies obtained in Example 3-7, and then 20 ml of 36% $Na_2SO_4$ was added to finally obtain a 18% $Na_2SO_4$ solution. The solution was centrifuged at 10,000 rpm for 15 minutes, and the supernatant was removed. The pellets were dialyzed against PBS buffer overnight, and then adsorbed on a column of Protein A Sepharose 4B. The column was eluted with acetate buffer (0.1M acetic acid, 0.14M NaCl, pH 5.0, 4.3). The resulting fractions were pooled.

Example 4

Production of monoclonal antibodies using HRBC-100 and HRGC-100 as immunization antigens

In accordance with the method described in Example 3, BALB/C mice were immunized with HRBC-100 or HRGC-100 as antigens instead of HRBC-300 or HRGC-300. Fused cells were prepared from the immunized spleen cells and mouse myeloma cells, and hybridomas were obtained by HAT selection. The hybridomas were screened by ELISA and the staining of the cancer tissue slice to select hybridoma producing the desired antibodies. The hybridomas were cloned by the limiting dilution method, and then the desired antibodies were produced. The antibodies had a molecular weight of about 160,000 daltones, and the class and subclasses were $IgG_1$ for HRBC-100, and $IgG_{2a}$ for HRGC-100.

Example 5

Diagnosis of human breast and gastric cancers

Monoclonal antibodies to the antigens HRBC-300 and HRGC-300 obtained by the method of Example 1 were each prepared into a solution having a concentration of 4 micrograms/ml with sodium carbonate buffer (0.05M $NaHCO_3$, 0.05M $Na_2CO_3$, pH 9.5). the solution was dividedly poured into the wells of a 96-well microtiter plate in an amount of 0.1 ml per well. After sealing it, the plate was left to stand at 37 °C for 3 hours and subsequently at 4 °C overnight.

On the next morning, the plate was well washed with PBS buffer containing 0.05% Tween 20 (to be referred to as Tween 20·PBS). Serum samples were taken from 10 breast cancer patients, 10 healthy persons and 8 gastric cancer patients, and diluted with PBS buffer to 10, 20, 40, 80, and 160 times respectively. Each dilution was dividedly poured into the wells of the 96-well microtiter plate in an amount of 0.1 ml per well, and incubated at 36 °C for 3 hours. The serum was removed, and the plate was well washed with Tween 20·PBS.

Each of horseradish peroxidase-conjugated anti-HRBC-100 antibody and horseradish peroxidase-conjugated anti-HRGC-100 antibody previously prepared and stored [prepared by the method of Nakane (P. K. Nakane and A. Kawaoi, J. H. Histochem. Cytochem., 22, 1084, 1974) was adjusted to a concentration of 5 micrograms/ml with PBS buffer containing 0.5% BSA (buffer prepared by using PBS containing 0.025% Tween 20 so that the concentration of horse serum albumin became 0.5%). 0.2 ml of the resulting solution was poured into each of the wells of the microtiter plate and incubated at 36 °C for 3 hours. The plate was well washed with Tween 20·PBS, and 0.1 ml of ortho-phenylenediamine dihydrochloride adjusted to 0.5 mg/ml with McIlvaine's buffer (pH 5.3) containing 0.005% of hydrogen peroxide was poured into each the wells of the microtiter plate, and incubated for 30 minutes.

Then, 0.05 ml of 0.4M sulfuric acid solution was added to each well to stop the reaction. The absorbance at 492 nm of each well was measured by a Corona 2-wave microplate photometer (made by Corona Electric Co., Ltd.). The absorbances of the serum samples of the 10 breast cancer patients, 8 gastric cancer patients and 10 healthy persons, diluted to 10 times, are shown in Figures 1 and 2. The absorbances of the serum samples from one breast cancer patient and three healthy persons (one of which was a pregnant woman in the 8th month of pregnancy who showed a relatively high absorbance) at various dilution ratios are shown in Figure 3. The foregoing demonstrates that the cancer patients and the healthy persons can be clearly distinguished, and the diagnosis kit for cancer using the antibodies in accordance with this invention is useful.

## Possibility of Utilization in Industry

As is clear from the Examples, the antigens HR-300 and HR-100 of this invention are cancer-specific. The use of these antigens easily gives antibodies which react specifically with various types of cancer. The Examples also demonstrate that a clear and accurate diagnosis of cancer is possible by utilizing the resulting cancer-specific antibodies, and led to the confirmation of the effects of this invention.

Hence, the human cancer-specific monoclonal antibodies produced by the process of this invention permit early discovery of antigens specific for cancer (particularly breast cancer and gastric cancer) from the blood and body fluids of patients, and provide physicians with guidelines for the diagnosis and treatment of the patients. It is also useful as means of monitoring relapse of patients who have been treated in the early stage.

## Scope of Claims

1. A process for producing a human cancer specific monoclonal antibody, which comprises centrifuging a finely crushed homogenized mixture of human cancer cells, centrifuging the supernatant in a discontinuous sucrose density gradient solution, collecting fractions which gather on the discontinuous phase of the density gradient, superimposing the fractions on a continuous sucrose density gradient solution, ultracentrifuging them to separate fractions rich in a cancer specific antigen having a density of 1.191ρ to 1.210ρ, immunizing an animal with the fractions as an antigen, preparing fused cells between spleen cells of the immunized animal and myeloma cells, screening the resulting hybridoma cells to select those hybridoma cells which produce the desired antibody, cloning the selected hybridoma cells, and then culturing the clones.

2. The process of claim 1 wherein the centrifugation of the finely crushed mixture is carried out at about 650 x g to about 1500 x g.

3. The process of claim 1 wherein the centrifugation in the discontinuous sucrose density gradient solution is carried out for 1 to 2 hours at about 60,000 x g to about 200,000 x g at a temperature below about 10 °C at which the solution is not frozen.

4. The process of claim 1 wherein ultracentrifugation on the continuous sucrose density gradient is carried out for at least 4 hours at about 60,000 x g to about 200,000 x g at a temperature below about 10 °C at which the solution is not frozen.

5. The process of claim 1 wherein the human cancer is breast cancer.

6. The process of claim 1 wherein the human cancer is gastric cancer.

7. The process of any one of claims 1 to 6 wherein the antigen set forth in claim 1 is treated with a carbohydrase to remove its carbohydrate portion.

FIGURE 1

DISTRIBUTION OF THE ABSORBANCES OF THE SERA OF BREAST CANCER
PATIENTS AND HEALTHY PERSONS AT A DILUTION RATIO OF 10

DILUTED TO 10 TIMES

FIGURE 2

DISTRIBUTION OF THE ABSORBANCES OF THE SERA OF GASTRIC CANCER
PATIENTS AND HEALTHY PERSONS AT A DILUTION RATIO OF 10

0207170

FIGURE 3

DISTRIBUTION OF THE ABSORBANCES OF THE SERA OF
A BREAST CANCER PATIENT AND HEALTHY PERSONS AT
VATIOUS DILUTION RATIOS

Δ BREAST CANCER
  PATIENT

X HEALTHY PERSONS
  (8 MONTH-PREGNANT WOMAN)

O HEALTHY PERSON

□ HEALTHY PERSON

0207170

FIGURE 4

0207170

FIGURE 5

0207170

# INTERNATIONAL SEARCH REPORT

International Application No. PCT/JP85/00718

**I. CLASSIFICATION OF SUBJECT MATTER** (if several classification symbols apply, indicate all) ³

According to International Patent Classification (IPC) or to both National Classification and IPC

Int.Cl⁴ C12P 21/00, C12N 15/00, G01N 33/574, A61K 39/395 //
(C12P 21/00, C12R1:91), (C12N 15/00, C12R1:91)

**II. FIELDS SEARCHED**

| Minimum Documentation Searched ⁴ | |
|---|---|
| Classification System | Classification Symbols |
| IPC | C12P 21/00, C12N 15/00, G01N 33/574, G01N 33/577, A61K 39/395 |

Documentation Searched other than Minimum Documentation
to the Extent that such Documents are Included in the Fields Searched ⁵

**III. DOCUMENTS CONSIDERED TO BE RELEVANT** ¹⁴

| Category* | Citation of Document, ¹⁶ with indication, where appropriate, of the relevant passages ¹⁷ | Relevant to Claim No. ¹⁸ |
|---|---|---|
| A | JP, A, 59-176298 (Sloan Kettering Institute For Cancer Research), 5 October 1984 (05. 10. 84) Columns 1 to 3, columns 13 to 20 & EP, A2, 119556 | 1 - 7 |
| T | JP, A, 61-43200 (Nippon Kayaku Co., Ltd.), 1 March 1986 (01. 03. 86) Columns 12 to 17 (Family: none) | 1 - 7 |

\* Special categories of cited documents: ¹⁵

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search ² | Date of Mailing of this International Search Report ² |
|---|---|
| March 15, 1986 (15. 03. 86) | March 31, 1986 (31. 03. 86) |
| International Searching Authority ¹ | Signature of Authorized Officer ²⁰ |
| Japanese Patent Office | |

Form PCT/ISA/210 (second sheet) (October 1981)